# EUROPEAN PATENT APPLICATION

(11) **EP 3 629 274 A1**
(43) Date of publication of application: **01.04.2020**
(21) Application number: 18196599.7
(22) Date of filing: 25.09.2018
(51) Int. Cl.: G06Q 30/00, G16H 40/20

(54) **SMART CONTRACT BASED ORDERING OF MEDICAL PROCEDURES**

(71) Applicant: Siemens Healthcare GmbH, 91052 Erlangen (DE)
(72) Inventor: Popescu, Stefan, 91056 Erlangen (DE)

(57) **Abstract**

The invention relates to a computer-implemented method and a system for providing an offer dataset, comprising receiving a procedure specification related to a medical procedure from a payer entity, wherein the procedure specification comprises a procedure code of the medical procedure. The method furthermore comprises identifying a requested medical apparatus capable for executing the medical procedure based on the procedure code. The method furthermore comprises determining a procedure-specific technical parameter of the medical apparatus based on the medical procedure and/or based on the procedure code. The method furthermore comprises determining the offer dataset based on the procedure-specific technical parameter, wherein the offer dataset is an offer dataset for the medical procedure, and wherein the offer dataset comprises a smart contract. The method furthermore comprises providing the offer dataset to the payer entity, comprising documenting the offer dataset within a distributed ledger.

The invention furthermore relates to a computer-implemented method and a system for providing an order dataset, comprising optionally receiving an identifier of a patient, receiving an procedure code of a medical procedure to be executed, determining at least one requested provider entity capable for executing the medical procedure based on the procedure code, receiving at least one offer dataset based on the procedure code, wherein each of the at least one offer dataset is related to a provider entity of the at least one requested provider entities, determining a selected provider entity of the at least one requested provider entities based on the at least one offer dataset, determining the order dataset comprising the procedure code, the order dataset optionally comprising the identifier of the patient, providing the order dataset to the selected provider entity.

## Description

In a lot of world-wide countries, costs of or payments for medical procedures are not a result of supply and demand, but established by a central authority, for example by the government, a governmental agency or by an organization of medical practitioners (e.g. the "American Medical Association" in the United States, acronym "AMA", or the "Gemeinsamer Bundesausschuss" in Germany).

These central authorities use certain codes for medical procedures, in order to set up a standard framework for reimbursing the costs of these medical procedures. In the United States, these codes are called CPT (acronym for "Current Procedural Terminology") codes, which describe medical procedures (including imaging and related radiologic services) performed by physicians and other qualified health care professionals. These codes are then used by "Centers for Medicare and Medicaid" (acronym "CMS") for reimbursement to Medicare providers. Another known standard framework "International Statistical Classification of Diseases and Related Health Problems" (acronym "IDS").

Each year, the AMA updates the list of CPT codes by adding new codes and revising or deleting certain existing codes. CMS reviews this updated information and assigns "Relative Value Units" (an acronym is RVU) based on its analysis of the labor and resource input costs to each newly identified CPT code. Once CMS makes its RVU information available, healthcare providers uses this data to establish new rates for these new CPT codes.

The current use of these coding schemes has the disadvantage that a central authority is necessary for reimbursement of medical procedures. Furthermore, using standard (or flatrate) reimbursement of medical procedures across a whole region or country does not take into account provider-specific advantages or disadvantages (e.g. a high imaging quality by a new and costly imaging device, or an efficient management of the hospital resources allowing a higher number of daily patients per medical practitioner or per medical device).

So it is the problem of this invention to provide a more flexible and cost-efficient scheme for the reimbursement of medical procedures, which can take into account economical and technical differences of the medical devices of the different medical providers.

The problem is solved by a method for providing an offer dataset, a method for providing an order dataset, an offer providing system, an order providing system, a computer program and a computer-readable medium according to the independent claims. Improvements and advantageous embodiments are given in the dependent claims and in the description.

In the following the solution according to the invention is described with respect to the claimed systems as well as with respect to the claimed methods. Features, advantages or alternative embodiments herein can be assigned to the other corresponding claimed objects and vice versa. In other words, the systems can be improved with features described or claimed in the context of the corresponding methods. In this case, the functional features of the methods are embodied by objective units of the systems.

According to a **first aspect** the invention relates to a computer-implemented method for providing an offer dataset, comprising receiving a procedure specification related to a medical procedure from a payer entity, wherein the procedure specification comprises a procedure code of the medical procedure. The method furthermore comprises identifying a requested medical apparatus capable for executing the medical procedure based on the procedure code. The method furthermore comprises determining a procedure-specific technical parameter of the requested medical apparatus based on the medical procedure and/or based on the procedure code. The method furthermore comprises determining the offer dataset based on the procedure-specific technical parameter, wherein the offer dataset is an offer dataset for the medical procedure, and wherein the offer dataset comprises a smart contract. The method furthermore comprises providing the offer dataset to the payer entity, comprising documenting the offer dataset within a distributed ledger.

In particular, the step of receiving and/or the step of providing can be executed with an interface, in particular with an interface of an offer providing system. In particular, the step of identifying and/or the steps of determining can be executed with a computation unit, in particular with a computation unit of the offer providing system. In particular, the offer providing system can be located within and/or owned by and/or in communication with and/or related to a requested provider entity and/or the owner of the requested medical apparatus.

In particular, the step of receiving a procedure specification related to a medical procedure from a payer entity can be identical with or comprise the step of receiving the procedure specification related to the medical procedure from an order providing system. In particular, the step of providing the offer dataset to the payer entity can be identical with or comprise the step of providing the offer dataset to the order providing system. In particular, the order providing system can be located within and/or owned by and/or in communication with and/or related to the payer entity.

A **medical procedure** is a diagnostic and/or therapeutic procedure that is related to a human or animal patient. Examples for a diagnostic procedure are laboratory diagnostics based on a sample of the patient (blood, liquor, liquids, etc.), or a medical imaging of the patient (e.g. by means of magnetic resonance imaging, computed tomography, X-ray imaging or ultrasound imaging). Examples for therapeutic procedures are drug administration (e.g. by an apparatus for drug infusion) or executing a surgical operation (e.g. by means of an assisting robot). A **medical apparatus** is a technical apparatus executing a medical procedure or a technical apparatus used for executing a medical procedure. In particular, for executing the medical procedure, an operator can control the medical apparatus. In particular, the requested medical apparatus is a medical apparatus.

In general an **entity** can be a person, a group of person, a legal entity (such as a company), a group of legal entities, or an object, as a server, a client, or a computer. In general, a **provider entity** is an entity executing medical procedures; in particular, an provider entity can be a hospital, a medical practice, a doctor's office or any group thereof. Furthermore a provider entity can also be a medical research facility or a medical researcher. In particular, the requested provider entity is a provider entity. In general, a **payer entity** is an entity requesting and/or reimbursing a medical procedure. In particular, a payer entity can be the patient being subject of the medical procedure, a physician or a hospital ordering a medical procedure which it is not capable of performing itself, or an insurance company, in particular a health insurance company.

In general, an **order dataset** is a dataset which can be received by a provider entity specifying a future medical procedure to be executed. The order dataset can comprise further details, for example, the order dataset can be related to the patient being subject of the medical procedure, to a time before and/or after the medical procedure has to be executed, or to other parameters relevant for the medical procedure. In particular, the order dataset can be created by a payer entity and/or by the patient being subject of the medical procedure. As a synonym for "order dataset" the terms "order data" or "order information" can be used.

In general, an **offer dataset** is a dataset provided by a provider entity related to a future medical procedure to be executed. In particular, the offer dataset can comprise the reimbursement which needs to be payed to the provider entity before or after the future medical procedure will have been executed. The offer dataset can comprise further data, for example parameters related to the technical properties of the apparatus executing the future medical procedure, or a time before and/or after the medical procedure can be executed. In particular, the offer dataset can furthermore comprise data about the provider entity being the owner of the medical apparatus and/or creating the offer dataset. In particular, the offer dataset is provided to a payer entity and/or to the patient. As a synonym for "offer dataset" the terms "offer data" or "offer information" can be used.

In general, a **distributed ledger** is a certain type of a decentralized database. In particular, the distributed ledger is distributed in the sense that there are several copies of (at least parts of) the distributed ledger in different memory units, wherein the memory units are spatially or geographically distributed. The distributed ledger comprises multiple records, wherein the multiple records can be identified with database entries. In particular, the multiple records are organized as data blocks. In particular, the records are created by different entities, in particular different nodes of a network, and stored with the different entities, in particular within the different nodes of the records. In other words, the construction and maintenance of the records is typically not performed by a central authority, but independently by nodes of the network. In typical cases, each node of the network maintains a local copy of the distributed ledger.

In general, updating the distributed ledger is typically based on a consensus mechanism, wherein a consensus mechanism ensures that the different copies of the distributed ledger match, also in the cases of a delayed communication between the entities storing the copies of the distributed ledger.

In particular, documenting a smart contract in the distributed consent ledger is executed by storing the smart contract in the distributed ledger, in particular by storing program code or machine code of the smart contract in the distributed ledger. Alternatively, a hash of the (program code or the machine code of the smart contract) or a link to the smart contract can be stored in the distributed ledger.

In particular, storing data in a distributed ledger can comprise appending a further data block to the distributed ledger, wherein the further data block comprises the data to be stored. Appending a further data block to the distributed ledger can comprise executing the consensus mechanism, wherein executing the consensus mechanism can comprise a proof of work, a proof of storage, a proof of stake, and/or a proof of elapsed time. A proof of work can be a compute-bound proof of work, a network-bound proof of work and/or a memory-bound proof of work.

In general, a **smart contract** comprises program elements which can be executed by a server, a client, or any other computation node. In particular, the program elements can comprise program logic, source code, scripting language and/or compiled machine code. In particular, a smart contract is documented in a distributed ledger, in particular in a distributed ownership ledger or in a distributed consent ledger. In particular, a smart contract can comprise conditions in terms of program logic, and consequences in terms of program logic, wherein the consequences are activated or executed if certain conditions are fulfilled.

The inventor recognized that by the proposed method for providing an offer dataset no intermediary central authority is necessary for reimbursing the medical procedure. In particular, by using a smart contract documented in a distributed ledger, the smart contract is non-deniable and non-disputable, and can be executed automatically if the conditions of the smart contract are fulfilled.

Furthermore, by the offer dataset being based on the procedure-specific technical parameter of the medical apparatus, specific technical properties of the medical apparatus can be considered for determining the offer dataset, and in particular, the offer dataset can be specifically adapted for the capabilities, the capacity and the costs of the medical apparatus.

According to a **further aspect** of the invention the procedure-specific technical parameter relates at least to one of a procedure time necessary for the requested medical apparatus executing the medical procedure, an amount of consumables needed by the requested medical apparatus for executing the medical procedure, and a number of operators necessary to execute the medical procedure with the requested medical apparatus. In particular, the procedure-specific technical parameter comprises at least one of the procedure time necessary for the requested medical apparatus executing the medical procedure, the amount of consumables needed by the requested medical apparatus for executing the medical procedure, and the number of operators necessary to execute the medical procedure with the requested medical apparatus.

The inventor recognized that by using one or more of these technical parameters the reimbursement and/or the offer dataset can be tailored to the technical properties of the requested medical apparatus executing the medical procedure.

According to a **further aspect** of the invention, the procedure-specific technical parameter is determined based on a database comprising at least one historic procedure-specific technical parameter of the requested medical apparatus executing a historic medical procedure related to the procedure code of the medical procedure. In particular, the procedure-specific technical parameter can be the last of the at least one historic procedure-specific technical parameter stored in the database, alternatively, the procedure-specific technical parameter can be a weighted or non-weighted average of the at least one historic procedure-specific technical parameter.

The inventor recognized that the procedure-specific technical parameter can be determined based on at least one historic procedure-specific technical parameter with a high accuracy.

According to a **further aspect** of the invention the method for providing the offer dataset furthermore comprises the step of receiving from the payer entity an order dataset comprising an procedure code of a medical procedure and an identifier of a patient, the step of identifying the patient based on the identifier of the patient, the step of determining a result of the medical procedure by executing the medical procedure on the patient with the selected medical apparatus, and the step of providing the result of the medical procedure to the payer entity.

In particular, the step of receiving and/or the step of providing can be executed with an interface, in particular with an interface of an order execution system. In particular, the step of identifying can be executed with a computation unit, in particular with a computation unit of the order execution system. In particular, the order execution system can be located within and/or owned by and/or in communication with and/or related to a selected provider entity and/or the owner of the selected medical apparatus.

The selected provider entity can be identical with the requested provider entity; alternatively the selected provider entity and the requested provider entity are different. The order execution system can be identical with the offer providing system; alternatively the order execution system and the offer providing system are different. The selected medical apparatus can be identical with the requested medical apparatus; alternatively the selected medical apparatus and the requested provider medical apparatus are different.

In particular, if the selected provider entity is identical with the requested provider entity, the order execution system is identical with the offer providing system and the selected medical apparatus is identical with the requested medical apparatus, and vice versa. In particular, if the selected provider entity is different from the requested provider entity, the order execution system is different from the offer providing system and the selected medical apparatus is different from the requested medical apparatus, and vice versa.

In particular, the step of receiving from the payer entity an order dataset can be identical with or comprise the step of receiving from the order providing system an order dataset. In particular, the step of providing the result of the medical procedure to the payer entity can be identical with or comprise the step of providing the result of the medical procedure to the order providing system.

The presence of the step of providing the result of the medical procedure to the payer entity is not essential for the method and can be considered to be an optional step.

An **identifier of the patient** can generally be used to unambiguously identify the patient. In particular, the identifier of the patient can comprise the name of the patient, the data of birth of the patient and/or an ID of the patient (e.g. the social insurance number, the ID number, a driving license number, or a passport number). In particular, the identifier of the patient can comprise an identifier of a device used by the patient, e.g. the IP (acronym for "Internet Protocol") address , the IMEI (acronym for "International Mobile Equipment Identity"), the MAC (acronym for "Media Access Control)" address of the device (e.g. a computer or a mobile device) used by the patient. In particular, the identifier of the patient can be based on a public key or on a private key related with the patient. In particular, the identifier of the patient can also be a shared secret or token. The term "identifier of the patient" is equivalent to the term "patient identifier". Furthermore, instead of the term "identifier of the patient" the short term "identifier" can be used.

For identifying the patient based on the identifier, it is determined whether a person impersonating the patient is in fact identical with the person. This can be done automatically, for example by interacting with a technical device of the person.

In particular, for providing the result of the medical procedure to the payer entity, the result of the medical procedure can be directly sent to the payer entity, or the result of the medical procedure can be provided so that it can be accessed by the payer entity, for example uploaded to a server, or documented in a distributed ledger. If the result of the medical procedure is available to the public, the result of the medical procedure can be encrypted or de-identified.

The inventor recognized that by using an identifier of the patient to identify the patient it can be ensured that the medical procedure to be executed is actually ordered for the respective patient, avoiding the execution of wrong medical procedures. Furthermore, by automatically providing the result of the medical procedure to the payer entity the result of the medical procedure can be accessed faster by the payer entity.

According to a **further aspect** of the invention the patient identifier is based on a public key corresponding to a private key, wherein the private key is stored on a key device of the patient, and wherein the identifying the patient is based on an interaction with the key device of the patient. In particular, the interaction can be based on a challenge-response procedure. In particular, the patient identifier is identical with the private key.

The inventor recognized that by using an asymmetric encryption based on a public and private key the identification of the patient can be executed without communicating and/or revealing the identifier of the patient to the public.

According to a **further aspect** of the invention the method furthermore comprises providing a digital signature to the payer entity, wherein the digital signature is signed with the private key of the patient. In particular, the digital signature was determined on the patient key device. In particular, the step of providing can be executed with the interface, in particular with the interface of the order execution system.

In general a **digital signature** relies on an asymmetric key pair comprising a private key and a public key, originating from an asymmetrical cryptography system. A public key is corresponding with a private key or vice versa, if the private key and the public key form an asymmetric key pair. In particular, a digital signature is the output of a signing algorithm, wherein the signing algorithm takes as first input the data to be signed (also denoted as "message") and as second input a signing information. In particular, the signing information is the private key of the patient. In particular, a signature is signed with the private key of the patient, if said private key was used as second input for the signing algorithm when creating said signature. In particular, a signature is based on certain data, if said certain data was used as first input for the signing algorithm when creating said signature.

In particular, for a signing algorithm there exists a signature verifying algorithm, wherein the signature verifying algorithm takes as input a dataset, a signature and a public key, and wherein the output of the signature verifying algorithm determines whether the given signature is the output of the signing algorithm applied to the dataset with a private key corresponding to the given public key. In particular, the signature verifying algorithm can verify the validity of a signature without the knowledge of the private key corresponding to the public key. In particular, a private key used to sign a signature cannot be deduced from said signature.

In particular, the digital signature can be based on at least one of the order dataset and the result of the medical procedure. Furthermore, the digital signature can additionally be based on other data.

The inventor recognized that by providing a signature signed with the private key of the patient to the payer entity a proof can be submitted to the payer entity that the medical procedure actually has been performed. This increases the reliability of the reimbursement system.

According to a **further aspect** of the invention, the smart contract regulates the transfer of cryptocurrency if a result of the medical procedure is provided to the payer entity, wherein the cryptocurrency is transferred from a first account corresponding to the payer entity to a second account corresponding to a provider entity. According to a further possible aspect of the invention, the smart contract regulates the transfer of cryptocurrency if the digital signature is provided to the payer entity, wherein the cryptocurrency is transferred from a first account corresponding to the payer entity to a second account corresponding to the provider entity. According to a further possible aspect of the invention, the smart contract regulates the transfer of cryptocurrency if the digital signature and the result of the medical procedure are provided to the payer entity, wherein the cryptocurrency is transferred from a first account corresponding to the payer entity to a second account corresponding to the provider entity. In particular, the provider entity is the selected provider entity.

In particular, the providing of the result of the medical procedure and/or the providing of the digital signature can be based on documenting the result of the medical procedure and/or the digital signature in the distributed ledger. In particular, documenting the result of the medical procedure in the distributed ledger can be executed by documenting a hash value of the result of the medical procedure in the distributed ledger.

In general a **cryptocurrency** is a digital asset or a digital medium which can be exchanged between different entities, wherein methods of cryptography are used for transferring cryptocurrencies from a first entity to a second entity, for the creation of a new amount of cryptocurrency and/or for verifying transfers of cryptocurrency from a first entity to a second entity. As an alternative term for "cryptocurrency" the term "virtual currency" can be used. In particular, a cryptocurrency is a digital currency. Cryptocurrency can be stored in accounts (another term is "wallet"), in particular, an account corresponds to a asymmetric key pair comprising a private key and a public key, wherein the private key must be used for transferring cryptocurrency from said account to another account, and wherein the public key must be used for transferring cryptocurrency from another account to said account. In particular, an account can be identified by the public key.

In general, **transferring a certain amount of cryptocurrency** from a first account to a second account comprises creating a transaction log, wherein the first account corresponds to a asymmetric key pair comprising a first private key and a first public key, and wherein the second account corresponds to an asymmetric key pair comprising a second private key and a second public key, and wherein the transaction log comprises the second public key and at least one key of the first private key and the first public key, and wherein the transaction log furthermore comprises the amount of cryptocurrency being transferred. In particular, a transaction log can also comprise a signature based on the amount of cryptocurrency being transferred and the second public key, signed with the first private key. In particular, transferring a certain amount of cryptocurrency is executed by including the transaction log into a data block of the distributed ledger or another distributed ledger.

In particular, the selected **provider entity** is the owner of the medical apparatus or the entity which executed the method for providing an offer dataset.

The inventors recognized that by the transfer of cryptocurrency being regulated by the smart contract, the reimbursement process between the payer entity and the provider entity can be automatized. In particular, the reimbursement can be done automatically in reaction to the providing of the result of the medical procedure and/or the digital signature, without involving any intermediary.

According to **a second aspect** the invention relates to a computer-implemented method for providing an order dataset, comprising optionally receiving an identifier of a patient, receiving an procedure code of a medical procedure to be executed, determining at least one requested provider entity capable for executing the medical procedure based on the procedure code, receiving at least one offer dataset based on the procedure code, wherein each of the at least one offer dataset is related to a provider entity of the at least one requested provider entities, determining a selected provider entity of the at least one requested provider entities based on the at least one offer dataset, determining the order dataset comprising the procedure code, the order dataset optionally comprising the identifier of the patient, providing the order dataset to the selected provider entity.

In particular, the steps of receiving and/or the step of providing can be executed with an interface, in particular with an interface of an order providing system. In particular, the steps of determining and the step of receiving at least one offer dataset can be executed with a computation unit, in particular with a computation unit of the order providing system. In particular, the order providing system can be located within and/or owned by and/or in communication with and/or related to a payer entity.

In particular, the step of providing the order dataset to the selected provider entity can be identical with or comprise the step of providing the order dataset to an offer providing system. In particular, the offer providing system can be located within and/or owned by and/or in communication with and/or related to the requested provider entity.

The inventor recognized that by this second aspect a suitable provider entity or medical apparatus for performing the medical procedure can be detected by the payer entity. Furthermore, by providing the order dataset to the provider entity the provider entity can make arrangements for executing the medical procedure, such as scheduling the medical apparatus, ordering consumables, or adjust parameters of the medical apparatus executing the medical procedure.

According to a **further aspect** of the invention, within the method for providing an order dataset, the at least one offer dataset is documented in a distributed ledger, and wherein receiving the at least one offer dataset comprises querying the distributed ledger.

The inventor recognized that by the at least one offer datasets being documented in a distributed ledger, the at least one offer datasets are immutable and non-disputable, which increases the reliability and the security of the system. Furthermore, interacting with the provider entities, in particular with at least one offer datasets from the provider entities, can be done automatically without a human operator. According to a **further aspect** of the invention, within the method for providing an order dataset, the at least one offer datasets a smart contract.

The inventor recognized that by the at least one offer dataset comprising a smart contract, the reimbursement process can be simplified. In particular, if executing the medical procedure based on the order dataset, a payment can be established by means of the smart contract as a reaction to a predefined conditions being true (e.g. the result of the medical procedure being made available to the payer entity), without a human to intervene.

According to a **third possible aspect** the invention relates to a method for exchanging an order dataset and an offer dataset, the method comprising:
- optionally receiving an identifier of a patient with an order providing system,
- receiving an procedure code of a medical procedure to be executed with the order providing system,
- determining at least one requested provider entity capable for executing the medical procedure based on the procedure code with the order providing system,
- transmitting a procedure specification from the order providing system to an offer providing system,
   wherein the procedure specification comprises the procedure code, and wherein the offer providing system is related to a requested provider entity of the at least one requested provider entities,
- identifying a requested medical apparatus capable for executing the medical procedure based on the procedure code with the offer providing system,
- determining a procedure-specific technical parameter of the requested medical apparatus based on the medical procedure and/or the procedure code with the offer providing system,
- determining the offer dataset based on the procedure-specific technical parameter with the offer providing system, wherein the offer dataset comprises a smart contract,
- transmitting the offer dataset from the offer providing system to the order providing system, comprising documenting the offer dataset within a distributed ledger,
- receiving at least one offer dataset based on the procedure code with the order providing system,
   wherein each of the at least one offer datasets is related to a provider entity of the at least one provider entities, wherein the at least one offer dataset comprises the offer dataset,
- determining a selected provider entity of the at least one provider entities based on the at least one offer dataset with the order providing system,
- determining the order dataset comprising the procedure code with the order providing system, the order dataset optionally comprising the identifier of the patient,
- providing the order dataset to the selected provider entity with the order providing system.

According to a **fourth aspect** the invention relates to an offer providing system for providing an offer dataset, comprising:
- an interface, configured for receiving a procedure specification related to a medical procedure from a payer entity, wherein the procedure specification comprises an procedure code of the medical procedure,
   furthermore configured for providing the offer dataset to the payer entity, comprising documenting the offer dataset within a distributed ledger,
- an calculation unit, configured for identifying a requested medical apparatus capable for executing the medical procedure based on the procedure code,
furthermore configured for determining a procedure-specific technical parameter of the requested medical apparatus based on the medical procedure and/or based on the procedure code, furthermore configured for determining the offer dataset based on the procedure-specific technical parameter, wherein the offer dataset is an offer dataset for the medical procedure, and wherein the offer dataset comprises a smart contract.

In particular, the offer providing system for providing an offer dataset can be configured to execute the method for providing an offer dataset according to the invention and its aspects. The offer providing system is configured to execute the method and its aspects by the interface and the computation unit being configured to execute the respective method steps.

According to a **fifth aspect** the invention relates to an order providing system for providing an order dataset, comprising:
- an interface, optionally configured for an identifier of a patient,
   furthermore configured for receiving an procedure code of a medical procedure to be executed,
   furthermore configured for providing the order dataset to the selected provider entity,
- a computation unit, configured for determining at least one requested provider entity capable for executing the medical procedure based on the procedure code,
furthermore configured for receiving at least one offer dataset based on the procedure code, wherein each of the at least one offer dataset is related to a provider entity of the at least one requested provider entities,
furthermore configured for determining a selected provider entity of the at least one requested provider entities based on the at least one offer dataset,
furthermore configured for determining the order dataset comprising the procedure code, the order dataset optionally comprising the identifier of the patient.

In particular, the order providing system for providing an order dataset can be configured to execute the method for providing an order dataset according to the invention and its aspects. The order providing system is configured to execute the method and its aspects by the interface and the computation unit being configured to execute the respective method steps.

According to a **sixth aspect** the invention relates to a system for exchanging an order dataset and an offer dataset, comprising an offer providing system according to an aspect of the invention and an order providing system according to another aspect of the invention.

In particular, system for exchanging an order dataset and an offer dataset can be configured to execute the method for exchanging an order dataset and an offer dataset according to the invention and its aspects. The system for exchanging an order dataset and an offer dataset is configured to execute the method and its aspects by the offer providing system and the order providing system being configured to execute the respective method steps.

All systems can be realized as a data processing system or as a part of a data processing system. Such a data processing system can, for example, comprise a cloud-computing system, a computer network, a computer, a tablet computer, a smartphone or the like. The systems can comprise hardware and/or software. The hardware can be, for example, a processor system, a memory system and combinations thereof. The hardware can be configurable by the software and/or be operable by the software.

According to a further aspect the invention relates to a computer program (or computer program product) comprising program elements which induce an offer providing system to carry out the steps of the method for providing an offer dataset according to the invention and/or its aspects, when the program elements are loaded into a memory unit of the offer providing system, and/or comprising program elements which induce an order providing system to carry out the steps of the method for providing an order dataset according to the invention and/or its aspects, when the program elements are loaded into a memory unit of the order providing system.

According to a further possible aspect the invention relates to a computer program (or computer program product) comprising program elements which induce an offer providing system to carry out the steps of the method for providing an offer dataset according to the invention and/or its aspects, when the program elements are loaded into a memory unit of the offer providing system.

According to a further possible aspect the invention relates to a computer program (or computer program product) comprising program elements which induce an order providing system to carry out the steps of the method for providing an order dataset according to the invention and/or its aspects, when the program elements are loaded into a memory unit of the order providing system.

According to a further aspect the invention relates to a computer-readable medium on which program elements are stored that can be read and executed by an offer providing system, in order to perform steps of the method for providing an offer dataset according to the invention and/or its aspects, when the program elements are executed by the offer providing system, and/or on which program elements are stored that can be read and executed by an order providing system, in order to perform steps of the method for providing an order dataset according to the invention and its aspects, when the program elements are executed by the order providing system.

The realization of the invention by a computer program (product) and/or a computer-readable medium has the advantage that already existing systems can be easily adopted by software updates in order to work as proposed by the invention.

The computer program product can be, for example, a computer program or comprise another element apart from the computer program. This other element can be hardware, for example a memory device, on which the computer program is stored, a hardware key for using the computer program and the like, and/or software, for example a documentation or a software key for using the computer program.

The properties, features and advantages of this invention described above, as well as the manner they are achieved, become clearer and more understandable in the light of the following description and embodiments, which will be described in details in the context of the drawings. This following description does not limit the invention on the contained embodiments. Same components or parts can be labeled with the same reference signs in different figures. In general the figures are not for scale. In the following:
- Fig. 1: displays a data flow between the payer entity and the provider entity,
- Fig. 2: displays a flowchart of an embodiment of the method for providing an offer dataset,
- Fig. 3: displays a flowchart of an embodiment of the method for providing an order dataset,
- Fig. 4: displays a flowchart of an embodiment of the method for exchanging an offer dataset and an order dataset,
- Fig. 5: displays an embodiment of a distributed ledger,
- Fig. 6: displays an order providing system, an offer providing system and an order execution system.

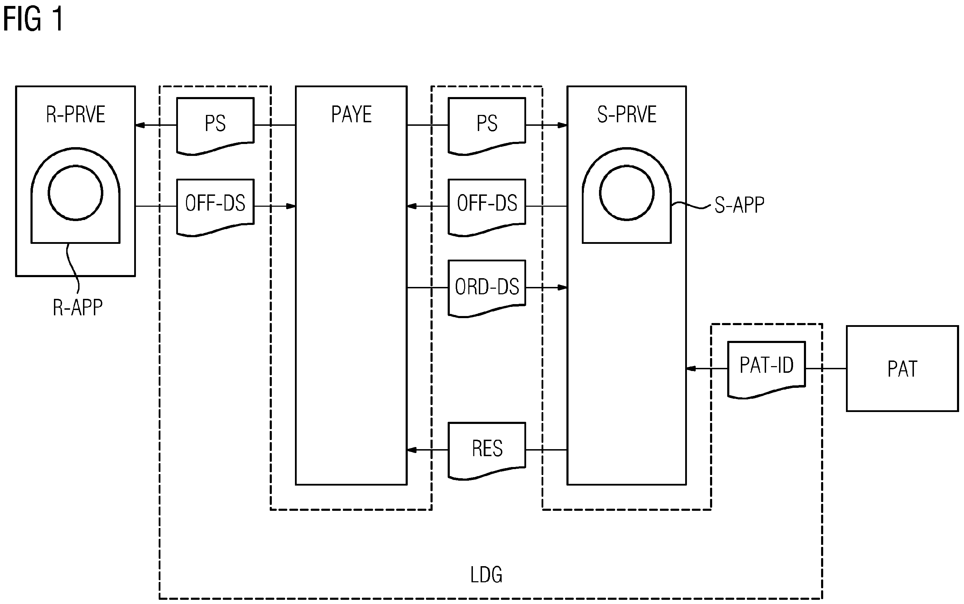

**Fig. 1** displays the data flow between the payer entity PAYE, the requested provider entity R-PRVE, the selected provider entity S-PRVE, and the patient PAT. In this embodiment, the patient PAT and the payer entity PAYE are different entities, alternatively the patient PAT and the payer entity PAYE can be the same or identical entity. Furthermore, in this embodiment the requested provider entity R-PRVE and the selected provider entity S-PRVE are different entities, alternatively the requested provider entity R-PRVE and the selected provider entity S-PRVE can be the same or identical entity.

In general, the starting situation is that the payer entity PAYE wants to order a certain medical procedure for a patient PAT, for example an examination with a computed tomography apparatus. Therefor the payer entity PAYE transmits a procedure specification PS, PS.1, ..., PS.3, to several provider entities R-PROVE, S-PRVE, the procedure specification PS, PS.1 ..., PS.3 comprising a CPT code for the computed tomography examination to be executed. The different provider entities R-PRVE, S-PRVE can react by sending offer datasets OFF-DS to the payer entity PAYE, the offer datasets OFF-DS comprising data which allows the payer entity PAYE to choose between the different offer datasets OFF-DS, for example procedure-specific technical parameters TP.1, TP.2 of the medical apparatuses R-APP, S-APP of the respective provider entities R-PRVE, S-PRVE. Based on these offer datasets OFF-DS, the payer entity PAYE decides on one of the provider entities R-PRVE, S-PRVE to actually execute the medical procedure on the patient PAT. The provider entity actually executing the medical procedure can be denoted as selected provider entity S-PRVE, whereas the other provider entities can be denoted as requested provider entities R-PRVE. In particular, all of the provider entities R-PRVE, S-PRVE which receive the procedure specification PS, PS.1 ..., PS.3 can be denoted as requested provider entities R-PRVE, and the one of the requested provider entities R-PRVE selected by the payer entity PAYE can be denoted as selected provider entity S-PRVE.

The payer entity PAYE, the requested provider entity R-PRVE, the selected provider entity S-PRVE and the patient PAT can exchange data and/or information directly or by means of an intermediary, in particular a distributed ledger LDG. The way of exchanging data and/or information can differ for different data and/or information being exchanged. For exchanging data and/or information by means of the distributed ledger LDG, the sending entity documents the data and/or information within the distributed ledger LDG, and the receiving entity accesses the distributed ledger LDG to read the documented data and/or information.

In this embodiment, the offer dataset OFF-DS and order dataset ORD-DS are exchanged by means of a distributed ledger LDG, and the procedure specification PS, PS.1 ..., PS.3 as well as the identifier PAT-ID of the patient PAT and the results RES of the medical procedure are exchanged directly without the distributed ledger LDG being involved. Alternatively, also the procedure specification PS, PS.1 ..., PS.3, the identifier PAT-ID of the patient PAT and the results RES of the medical procedure can be exchanged by means of the distributed ledger LDG. Alternatively, also the offer dataset OFF-DS and/or the order dataset ORD-DS can be exchanged directly without the distributed ledger LDG being involved.

In this embodiment, the offer dataset OFF-DS being exchanged by means of a distributed ledger LDG means that the offer dataset OFF-DS is documented within the distributed ledger LDG by the requested provider entity R-PRVE and/or the selected provider entity S-PRVE, and that the distributed ledger LDG is queried by the payer entity PAYE in order to receive the offer dataset OFF-DS. Furthermore in this embodiment, the order dataset ORD-DS being exchanged by means of a distributed ledger LDG means that the order dataset ORD-DS is documented within the distributed ledger LDG by the payer entity PAYE, and that the distributed ledger LDG is queried by the selected provider entity S-PRVE in order to receive the order dataset ORD-DS.

In this embodiment, the order dataset ORD-DS and/or the offer dataset OFF-DS is documented within the distributed ledger LDG if the order dataset ORD-DS and/or the offer dataset OFF-DS is contained in a data block DB.1, ..., DB.3 of the distributed ledger LDG. Alternatively, the order dataset ORD-DS and/or the offer dataset OFF-DS can be documented within the distributed ledger LDG by only a hash of the order dataset ORD-DS and/or a hash of the offer dataset OFF-DS being contained in a data block DB.1, ..., DB.3 of the distributed ledger LDG, wherein the order dataset ORD-DS and/or the offer dataset OFF-DS are exchanged directly or by using an intermediate entity (e.g. a server) between the payer entity PAYE and the requested provider entity R-PRVE or the selected provider entity S-PRVE.

Furthermore, in this embodiment the requested provider entity R-PRVE is the owner of a requested medical apparatus R-APP, and the selected provider entity S-PRVE is the owner of a selected medical apparatus S-APP. Both the requested medical apparatus R-APP and the selected medical apparatus S-APP are configured for executing the medical procedure specified in the procedure specification PS, PS.1 ..., PS.3.

Before executing the medical procedure, the person claiming to be the patient PAT should identify itself with respect to the selected provider entity S-PRVE or the selected medical apparatus S-APP. In this embodiment, the identification is done by sending a patient identifier PAT-ID from the patient PAT to the selected provider entity S-PRVE (or the selected medical apparatus S-APP) prior to executing the medical procedure. This patient identifier PAT-ID is then compared with a patient identifier PAT-ID being contained in the order dataset ORD-DS.

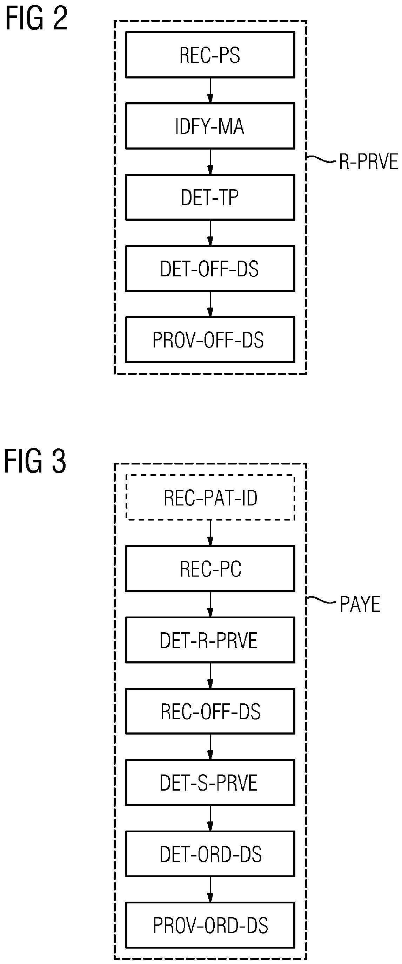

**Fig. 2** displays a flowchart of an embodiment of the method for providing an offer dataset ODD-DS.

The first step of the displayed embodiment is receiving REC-PS a procedure specification PS.1, ..., PS.3 related to a medical procedure from a payer entity PAYE, wherein the procedure specification PS.1, ..., PS.3 comprises an procedure code of the medical procedure. In this embodiment, the step of receiving REC-PS a procedure specification PS.1, ..., PS.3 is executed with an interface OFFS.IF. In particular, the interface OFFS.IF is an interface of the offer providing system OFFS.

In this embodiment, the procedure specification PS.1, ..., PS.3 is published by the payer entity PAYE and accessed by the requested provider entity R-PRVE or respectively the offer providing system OFFS related to the requested provider entity R-PRVE. In particular, the procedure specification PS.1, ..., PS.3 can be provided on a public server related to the payer entity PAYE, for example on a website of the payer entity PAYE or by a webservice of the payer entity PAYE. In this embodiment, the public server is not accessible by everyone, but only by a certain set of requested provider entities R-PRVE (which for example are certified by the payer entity PAYE); alternatively the public server can be accessible by everyone. Alternatively, it is also possible to directly send the procedure specification PS.1, ..., PS.3 from the payer entity PAYE to the requested provider entity R-PRVE, in particular, from an interface ORDS.IF of the payer entity PAYE or of an order providing system ORDS to the interface OFFS.IF of the requested provider entity R-PRVE or of the offer providing system OFFS, without an intermediary storage. Alternatively, the procedure specification PS.1, ..., PS.3 can be documented by the payer entity PAYE within a distributed ledger LDG, and the procedure specification PS.1, ..., PS.3 can be received by accessing the distributed ledger. In particular, the payer entity PAYE can send the procedure specification PS.1, ..., PS.3 to a plurality of requested provider entities R-PRVE.

In this embodiment, the procedure specification PS.1, ..., PS.3 comprises the procedure code and further data, for example a time-limit until or when the medical procedure has to be executed, or certain requirements that have to be fulfilled by the operators executing the medical procedure.

In particular, the payer entity PAYE, the requested provider entity R-PRVE and the selected provider entity S-PRVE are further connected to databases of governmental organizations and use the same procedure code (e.g. CPT codes or ICD-10 codes). Advantageously, the databases of governmental organizations can assign a standard code or a standard value of a code for certain medical procedures.

The second step of the displayed embodiment is identifying IDFY-MA a requested medical apparatus R-APP capable for executing the medical procedure based on the procedure code. In this embodiment, the second step of identifying IDFY-MA a requested medical apparatus R-APP is executed by a computation unit OFFS.CU. In particular, the computation unit OFFS.CU is a computation unit of the offer providing system OFFS.

In this embodiment, the procedure code is a CPT code (acronym for "Current Procedural Terminology"). For example, the CPT code "71250" corresponds to a CT (acronym for "computed tomography") scan of the chest without contrast agent, and the CPT code "71260" corresponds to a CT scan of the chest with contrast agent. In both cases, the requested medical apparatus R-APP should be capable of executing a computed tomography scan, in other words, the requested medical apparatus R-APP should be a computed tomography apparatus.

In this embodiment, identifying IDFY-MA the requested medical apparatus R-APP is done by querying a database, the database storing associations between the medical apparatuses available at the requested provider R-PROV, and the CPT codes they can be used for. So identifying IDFY-MA the requested medical apparatus R-APP is equivalent for searching the respective CPT in said database. Alternatively, it is possible to interact directly with the requested medical apparatus R-APP, for example by means of an API (acronym for "application programmable interface"), wherein the procedure code is sent to the requested medical apparatus R-APP, and wherein the requested medical apparatus R-APP responds whether it is possible for the requested medical apparatus R-APP to executed the (requested) medical procedure related to the procedure code.

The third step of the displayed embodiment is determining DET-TP a procedure-specific technical parameter TP.1, TP.2 of the requested medical apparatus R-APP based on the medical procedure and/or based on the procedure code. In this embodiment, the third step of determining DET-TP a procedure-specific technical parameter TP.1, TP.2 is executed by the computation unit OFFS.CU. In particular, the computation unit OFFS.CU is the computation unit of the offer providing system OFFS.

In this embodiment, the procedure-specific technical parameter TP.1, TP.2 can relates to least one of: the procedure time necessary for the requested medical apparatus R-APP executing the medical procedure; the amount of consumables needed by the requested medical apparatus R-APP for executing the medical procedure; and the number of operators necessary to execute the medical procedure with the requested medical apparatus R-APP. In particular, the procedure-specific technical parameter TP.1, TP.2 relates to these parameters by comprising said parameters. Alternatively, the procedure-specific technical parameter TP.1, TP.2 can relate to at least one of a subset of these parameters. Furthermore, the procedure-specific technical parameter TP.1, TP.2 can be related to additional technical parameters of the requested medical apparatus R-APP.

In this embodiment, the procedure-specific technical parameter TP.1, TP.2 is determined based on a database comprising at least one historic procedure-specific technical parameter of the requested medical apparatus R-APP executing a historic medical procedure related to the procedure code of the medical procedure. In particular, said database stores historic medical procedures executed by the requested medical apparatus R-APP, the procedure codes of the historic medical procedures and the associated historic procedure-specific technical parameters. The procedure-specific technical parameter TP.1, TP.2 is determined as an average of the last 10 (other numbers are of course possible) historic procedure-specific technical parameters of the requested medical apparatus R-APP. Alternatively, the median of a certain number of historic procedure-specific technical parameters of the requested medical apparatus R-APP can be used, or a weighted average of a number historic procedure-specific technical parameters of the requested medical apparatus R-APP.

As an optional step, if in the step of identifying IDFY-MA a requested medical apparatus R-APP several requested medical apparatuses R-APP are identified as capable for executing the medical procedure based on the procedure code, one requested medical apparatus R-APP can be determined from the identified set of medical apparatuses R-APP based on the procedure-specific technical parameters TP.1, TP.2 of each identified medical apparatus APP.

The fourth step of the displayed embodiment is determining DET-OFF-DS the offer dataset OFF-DS based on the procedure-specific technical parameter TP.1, TP.2, wherein the offer dataset OFF-DS is an offer dataset for the medical procedure, and wherein the offer dataset OFF-DS comprises a smart contract SC.1, SC.2. In this embodiment, the third step of determining DET-OFF-DS the offer dataset OFF-DS is executed by the computation unit OFFS.CU. In particular, the computation unit OFFS.CU is the computation unit of the offer providing system OFFS.

In particular, the smart contract SC.1, SC.2 regulates the transfer of cryptocurrency if a result RES of the medical procedure is provided to the payer entity PAYE or to the order providing system ORDS, wherein the cryptocurrency is transferred from a first account corresponding to the payer entity PAYE to a second account corresponding to the requested provider entity R-PRVE or the selected provider entity S-PRVE. In order to detect that the result RES of the medical procedure was provided to the payer entity PAYE, in the case the result RES of the medical procedure is documented within a distributed ledger LDG, the condition of the smart contract SC.1, SC.2 can related to the result RES of the medical procedure being available in the distributed ledger LDG. Alternatively, if the result RES of the medical procedure is provided directly to the payer entity PAYE, the condition of the smart contract SC.1, SC.2 can related to the result RES of the medical procedure being in an outbox of the requested provider entity R-PRVE or the selected provider entity S-PRVE, or the result RES of the medical procedure being in an inbox of the payer entity PAYE. Alternatively, of the result RES of the medical procedure is provided via a public server to the payer entity PAYE, the condition of the smart contract SC.1, SC.2 can related to the result RES of the medical procedure being accessible on said public server.

In particular, the smart contract SC.1, SC.2 regulates the transfer of a certain amount of cryptocurrency. The amount of cryptocurrency may correspond to the costs or the price to be charged for the medical procedure. In this embodiment, the amount of cryptocurrency determined based on the procedure-specific technical parameter TP.1, TP.2.

The transfer of cryptocurrency can be documented within the distributed ledger LDG used for documenting the smart contract SC.1, SC.2, but also other distributed ledgers LDG can be used for documenting the transfer of cryptocurrency.

The last step of the displayed embodiment is providing PROV-OFF-DS an offer dataset OFF-DS for the medical procedure to the payer entity PAYE, comprising documenting the offer dataset OFF-DS within a distributed ledger LDG. In this embodiment, the step of providing PROV-OFF-DS an offer dataset OFF-DS is executed with the interface OFFS.IF. In particular, the interface OFFS.IF is the interface of the offer providing system OFFS.

In this embodiment, the offer dataset OFF-DS is documented within the distributed ledger LDG, so that it can be accessed by the payer entity PAYE. For documenting the offer dataset OFF-DS within the distributed ledger LDG, a data block DB.1, ..., DB.3 comprising the offer dataset OFF-DS or certain data of the offer dataset OFF-DS has to be created and to be inserted into the distributed ledger LDG. Alternatively, the offer dataset OFF-DS can be transmitted directly to the payer entity PAYE, for example by means of an E-Mail or by using a software interface.

**Fig. 3** displays a flowchart of an embodiment of the method for providing an order dataset ORD-DS. In this embodiment, the payer entity PAYE is a physician wanting to order a computed tomography scan of his patient PAT, but not having a direct access to a computed tomography apparatus. Alternatively, the payer entity PAYE could also be an insurance company of the patient PAT, who has to be the subject of a future magnetic resonance tomography scan, and the payer entity PAYE wants to order a suitable magnetic resonance tomography procedure for a low price. Alternatively, the patient PAT can also be the subject of a future surgical procedure requiring a certain technical apparatus. This user stories or user scenarios are only for illustrational purposes, and do by no way restrict the description of the embodiment or the invention. In particular, the invention or the embodiment could be applied for arbitrary medical procedures.

The first step of the displayed embodiment is the optional step of receiving REC-PAT-ID an identifier PAT-ID of a patient PAT. In this embodiment, the step of receiving REC-PAT-ID an identifier PAT-ID of a patient PAT is executed with an interface ORDS.IF. In particular, the interface ORDS.IF is an interface of the order providing system ORDS.

In this embodiment, the payer entity PAYE receives the identifier PAT-ID of the patient PAT by using a technical device for reading the identifier PAT-ID stored within another technical key device of the patient PAT. For example, if the identifier PAT-ID is a shared secret, the patient PAT can create an encrypted message containing the identifier PAT-ID with an application on his smartphone, and the payer entity PAYE can access the shared secret by decrypting the message. In particular, encrypting and decrypting can be executed using an asymmetric encryption and decrypting algorithm. Alternatively, the identifier PAT-ID of the patient PAT can also be stored on a chip card of the patient PAT.

The second step of the displayed embodiment is receiving REC-PC a procedure code of a medical procedure to be executed. In this embodiment, the step of receiving REC-PC a procedure code is executed with the interface ORDS.IF. In particular, the interface ORDS.IF is the interface of the order providing system ORDS.

In this embodiment, the payer entity PAYE being an ordering physician enters the procedure code (being a CPC code) manually using keyboard being part of the interface ORDS.IF. Alternatively, there could be an automatic data transfer between a system managing an electronic health record or a hospital information system and the order providing system ORDS, in particular by means of the interface ORDS.IF of the order providing system ORDS. In particular if the payer entity PAYE is an insurance company, the procedure code can be received from a patient PAT asking for the reimbursement of a future medical procedure, wherein the patient PAT communicates with the insurance company by means of a website or a webservice.

Alternatively, instead of receiving REC-PC the procedure code of the medical procedure to be executed directly, the step of receiving REC-PC the procedure code may also comprise receiving a description of the medical procedure to be executed with the interface ORDS.IF, and the substep of determining a procedure code of the medical procedure to be executed based on the description of the medical procedure to be executed.

It is also possible that within the steps of receiving REC-PAT-ID the identifier PAT-ID of the patient PAT and/or of receiving REC-PC the procedure code the data is sent from the requested provider entity R-PRVE or the selected provider entity S-PRVE. By this alternative the requested provider entity R-PRVE or the selected provider entity S-PRVE can submit a proposal for executing a medical procedure, and by sending an order dataset ORD-DS, the payer entity PAYE can consent with the execution of the medical procedure.

The third step of the displayed embodiment is determining DET-R-PRVE at least one requested provider entity R-PRVE capable for executing the medical procedure based on the procedure code. In this embodiment, the step of determining DET-R-PRVE at least one requested provider entity R-PRVE is executed with a computation unit PRDS-CU. In particular, the computation unit ORDS.CU is a computation unit of the order providing system ORDS.

In this embodiment, for determining DET-R-PRVE at least one requested provider entity R-PRVE capable for executing the medical procedure based on the procedure code, a request is provided PROV-PC to all provider entities known to the payer entity PAYE (which can for example be stored in a database) whether they are capable of executing the medical procedure based on the procedure code, and the determining DET-R-PRVE is based on a response of the provider entities, in particular, the at least one requested provider entities R-PRVE are the provider entities with a positive response. In particular, the request and the response can be based on electronic communication, for example on e-mails, HTTP-requests and/or HTTP-responses (acronym for "Hypertext Transfer Protocol"), or interactions via APIs. Alternatively, the request and the response can be based on documenting and/or reading data within the distributed ledger LDG.

Alternatively, in this step all provider entities known to the payer entity PAYE can be determined DET-R-PRVE as capable for executing the medical procedure, and all provider entities known to the payer entity PAYE are requested provider entities R-PRVE. In this case, a requested provider entity R-PRVE not capable of executing the medical procedure can indicate this fact by not responding to this step or to the following steps of the method.

Alternatively, the step of determining DET-R-PRVE at least one requested provider entity R-PRVE can comprise interacting with a database, the database comprising an relationship between provider entities and medical procedures, in particular, for each provider entity of the provider entities within the database there is an assigned list of medical procedures, wherein the said provider entity is capable of executing each of medical procedures contained in the assigned list of medical procedures. In particular, the database can be an associative database, in particular comprising entries related to primary and/or secondary keys. In particular, the database can comprise a first table of provider entities, a second table of medical procedures, and a third table mapping the 1:N relationship between the provider entities and the medical procedures. Alternatively, the database can be related with the distributed ledger LDG, in particular, the database can be identical with the distributed ledger LDG. In particular, the at least one requested provider entities R-PRVE are the provider entities of the database to which a matching medical procedure is assigned.

The fourth step of the displayed embodiment is receiving REC-OFF-DS at least one offer dataset OFF-DS based on the procedure code, wherein each of the at least one offer datasets OFF-DS is related to a provider entity of the at least one requested provider entities R-PRVE. In this embodiment, the step of receiving REC-OFF-DS at least one offer dataset OFF-DS is executed with the computation unit ORDS.CU. In particular, the computation unit ORDS.CU is the computation unit of the order providing system ORDS. Alternatively, the step of receiving REC-OFF-DS at least one offer dataset OFF-DS can be executed with or together with the interface ORDS.IF. In particular, the interface ORDS.IF is the interface of the order providing system ORDS.

In this embodiment, each of the at least one requested provider entities R-PRVE documents an offer dataset OFF-DS within the distributed ledger LDG, so that the at least one offer datasets OFF-DS can be received REC-OFF-DS by querying or inspecting the distributed ledger LDG. Alternatively, the at least one requested provider entities R-PRVE can send the at least one offer datasets OFF-DS directly to the payer entity PAYE or the order providing system ORDS. Alternatively, the at least one requested provider entities R-PRVE can publish the at least one offer datasets OFF-DS, for example on a web-server or via a webservice.

The fifth step of the displayed embodiment is determining DET-S-PRVE a selected provider entity S-PRVE of the at least one requested provider entities R-PRVE based on the at least one offer dataset OFF-DS. In this embodiment, the step of determining DET-S-PRVE a selected provider entity S-PRVE is executed with the computation unit ORDS.CU. In particular, the computation unit ORDS.CU is the computation unit of the order providing system ORDS.

In particular, the at least one offer dataset OFF-DS comprise parameters on which the determining DET-S-PRVE of the selected provider entity S-PRVE can be based. In particular, the at least one offer dataset OFF-DS comprise at least one of the following parameters:
- costs for the medical procedure (in particular, the costs can be given in terms of a cryptocurrency), in particular, a smart contract SC.1, SC.2, and in particular, a smart contract SC.1, SC.2 regulating the transfer of a certain amount of cryptocurrency,
- appointment date for executing the medical procedure,
- technical parameters, in particular procedure-specific technical parameters TP.1, TP.2 of the requested medical apparatus R-APP possibly executing the medical procedure (in particular, technical parameters related to the quality of a result RES of the medical procedure).

In this embodiment, the at least one offer dataset OFF-DS comprises a technical parameter, in particular a procedure-specific technical parameter TP.1, TP.2 of the requested medical apparatus R-APP possibly executing the medical procedure, and the selected provider entity S-PRVE is determined based on comparing the technical parameters, in particular procedure-specific technical parameters TP.1, TP.2 of the several requested medical apparatuses R-APP, in order to select, choose or pick-up a selected provider entity S-PRVE which can provide the best quality result RES of the medical procedure.

Alternatively, the other parameters can be taken into account, too, for example the costs for the medical procedure, or the distance of place of residence of the patient PAT and the requested provider entity R-PRVE. In particular, a cost function can be defined taking into account the different parameters, and the determining DET-S-PRVE of the selected provider entity S-PRVE can comprise a minimization and/or maximization of the cost function.

In general, a technical parameter TP.1, TP.2 of the requested medical apparatus R-APP is a property of the medical apparatus given by the design or construction of the medical apparatus or adjustable by an operator. For example, if the requested medical apparatus R-APP is a magnetic resonance tomography apparatus, a possible technical parameter TP.1, TP.2 would be the magnet field strength of the main magnet, which is given by the design or construction of the requested medical apparatus R-APP. Another possible technical parameter TP.1, TP.2 would be the duration (or timespan) of the medical procedure, which is adjustable by the operator of the requested medical apparatus R-APP by selecting a certain scan protocol. As another example, if the requested medical apparatus R-APP is a robot assisting in a surgery, a possible technical parameter TP.1, TP.2 would be the precision of the robot, which is given by the design or construction of the requested medical apparatus R-APP.

The sixth step of the displayed embodiment is determining DET-ORD-DS the order dataset ORD-DS comprising the procedure code, the order dataset ORD-DS optionally comprising the identifier PAT-ID of the patient PAT. In this embodiment, the step of determining DET-ORD-DS the order dataset ORD-DS is executed with the computation unit ORDS.CU. In particular, the computation unit ORDS.CU is the computation unit of the order providing system ORDS.

The last step of the displayed embodiment is providing PROV-ORD-DS the order dataset ORD-DS to the selected provider entity S-PRVE. In this embodiment, the step of providing PROV-ORD-DS the order dataset ORD-DS is executed with the interface ORDS.IF. In particular, the interface ORDS.CU is the interface of the order providing system ORDS.

In this embodiment, the order dataset ORD-DS is provided PROV-ORD-DS to the selected provider entity S-PRVE by documenting the order dataset ORD-DS in the distributed ledger LDG. Advantageously the step of providing PROV-ORD-DS the order dataset ORD-DS can comprise informing the patient PAT-ID about the upcoming medical procedure to be executed.

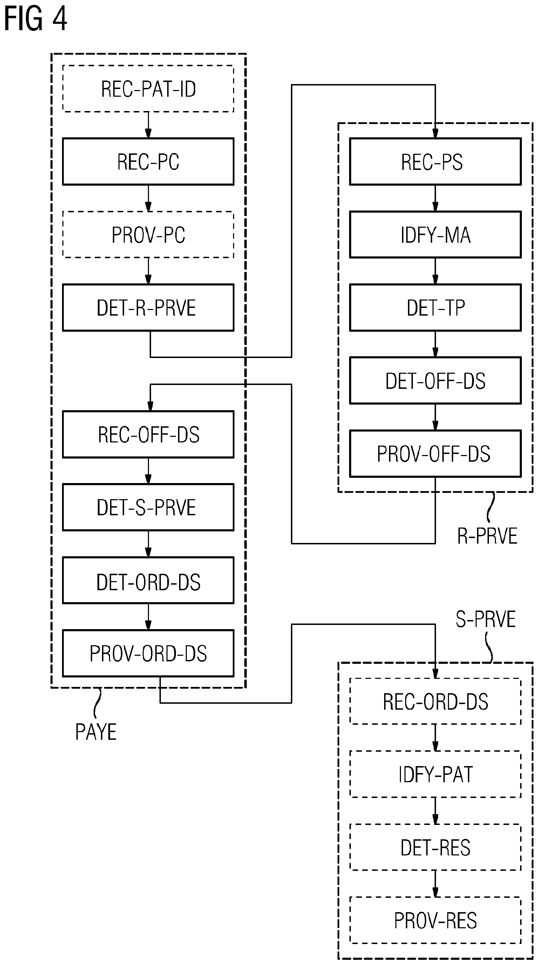

**Fig. 4** displays a flowchart of an embodiment of the method for exchanging an offer dataset OFF-DS and an order dataset ORD-DS. This embodiment can also be considered as embodiment of the method for providing an offer dataset PROV-OFF-DS and/or the method for providing an order dataset PROV-ORD-DS. The embodiment comprises steps already described in Fig. 2 and Fig. 3, all advantageous features, embodiments and alternatives described there can also be transferred to the steps within this embodiment.

The steps of the displayed embodiment are executed by the payer entity PAYE, the requested provider entity R-PRVE or the selected provider entity S-PRVE. Each step executed by the payer entity PAYE can also be considered as to be executed by the order providing system ORDS or one of its units (in particular, by the computation unit ORDS.CU and the interface ORDS.IF). Furthermore, each step executed by the requested provider entity R-PRVE can also be considered as to be executed by the offer providing system OFFS or one of its units (in particular, by the computation unit OFFS.CU and the interface OFFS.IF). Furthermore, each step executed by the selected provider entity S-PRVE can also be considered as to be executed by the order execution system OEXS or one of its units (in particular, by the computation unit OEXS.CU and the interface OEXS.IF).

The displayed embodiment furthermore comprises the optional step of receiving REC-ORD-DS the order dataset, in particular with the computation unit OEXS.CU and/or the interface OEXS.IF, in particular with the computation unit of the order execution system OEXS and/or the interface of the order execution system OEXS.

In this embodiment, the order dataset ORD-DS was provided PROV-ORD-DS by the interface ORDS.IF of the order providing system ORDS by documenting the order dataset ORD-DS within the distributed ledger LDG. In this case, the step of receiving REC-ORD-DS the order dataset ORD-DS comprises inspecting or accessing the distributed ledger LDG for determining a suitable order dataset ORD-DS. In particular, the order dataset ORD-DS can comprise an identifier of the selected provider entity S-PRVE or the order execution system OEXS. In particular, such an identifier can be a public key associated with the selected provider entity S-PRVE or the order execution system OEXS.

Alternatively, if the step of providing PROV-ORD-DS the order dataset is executed by directly transferring the order dataset ORD-DS to the selected provider entity S-PRVE, the step of receiving REC-ORD-DS the order dataset ORD-DS would comprise directly receiving the order dataset ORD-DS. Alternatively, if the step of providing PROV-ORD-DS the order dataset ORD-DS is executed by uploading the order dataset ORD-DS to a public server, the step of receiving REC-ORD-DS the order dataset ORD-DS would comprise accessing the public server.

The displayed embodiment furthermore comprises the optional step of identifying IDFY-PAT the patient PAT based on the identifier PAT-ID of the patient PAT, in particular with the computation unit OEXS.CU, in particular with the computation unit of the order execution system OEXS.

In this embodiment, the identifier PAT-ID of the patient PAT is a shared secret stored on a key device of the patient PAT, and furthermore contained in the order dataset ORD-DS. The step of identifying IDFY-PAT the patient PAT based on the identifier PAT-ID of the patient PAT comprises validating that the identifier PAT-ID stored in the key device and the identifier PAT-ID contained in the order dataset ORD-DS match. In particular, the key device of the patient PAT can send the identifier PAT-ID to the provider entity PRVE, or vice versa. Alternatively hashes of the identifier PAT-ID can be exchanged, so that the identifier PAT-ID is not transmitted as clear text.

Alternatively, the patient identifier PAT-ID can be based on a public key corresponding to a private key, wherein the private key is stored on a key device of the patient PAT, and the step of identifying IDFY-PAT the patient PAT is based on an interaction with the key device of the patient PAT. In particular, a message can be asymmetrically encrypted with the public key of the patient PAT, the asymmetrically encrypted message can be sent to the key device of the patient PAT. The key device can then asymmetrically decrypt the message with the private key. The knowledge of the message by the key device of the patient PAT then proofs that the key device is in possession of said private key, without exposing said private key.

As a key device, so called "cryptographic anchors" can be used, which can be embedded in everyday objects and devices. They are used in tandem with distributed ledgers LDG to ensure the authenticity of an object or a person whenever it interacts with the distributed ledger LDG. For example, a cryptographic anchor can be embedded into an armband, a patient PAT wristbands or hospital bracelet. Alternatively, a key device or a "cryptographic anchors" can be a so-called "blockchain phones", a smartphone configured to securely store and use digital coins (which are certain amounts of cryptocurrency) such as "Bitcoin" or other tokens, as well as related services, and possibly being configured to enable the identification, security of access control and distributed ledger LDG related functions and applications. In particular, a "blockchain phone" can store patient PAT health insurance data, as for example the standard electronic medical records. The "blockchain phone" phone may further store associated tokens to be used for daily health transactions. Users can verify their identities in order to use the "blockchain phone" with a face/iris scan, voice recognition, a fingerprint and/or a password.

Advantageously a positive and/or a negative identification of the patient PAT can also be recorded in the distributed ledger LDG, in order to document the occurrence of the identification process in a revision-safe way.

The displayed embodiment furthermore comprises the optional step of determining DET-RES a result RES of the medical procedure by executing the medical procedure on the patient PAT with the selected medical apparatus S-APP, and the optional step of providing PROV-RES the result RES of the medical procedure to the payer entity PAYE, in particular with the interface OEXS.IF. In particular, the interface OEXS.IF is the interface of the order execution system OEXS.

In this embodiment, the step of providing PROV-RES the result RES comprises documenting the result RES within the distributed ledger LDG. In particular, if the order dataset ORD-DS comprises a smart contract SC.1, SC.2, by documenting the result RES within the distributed ledger LDG a transaction of cryptocurrency can be initiated.

Advantageously, additional to the result RES a digital signature SGN is provided to the payer entity PAYE with the interface OEXS.IF of the order execution system OEXS, wherein the digital signature SGN is signed with the private key of the patient PAT. In particular, the digital signature SGN is a signature of the result RES of the medical procedure.

Advantageously, the step of providing PROV-RES the result RES can be executed after the transaction of cryptocurrency regulated in the smart contract SC.1, SC.2 has taken place. In this case, the trigger for the smart contract SC.1, SC.2 can be the providing of the digital signature SGN to the payer entity PAYE. In particular, the selected provider entity S-PRVE or the selected medical apparatus S-APP can provide access to the result RES only after the transaction of cryptocurrency.

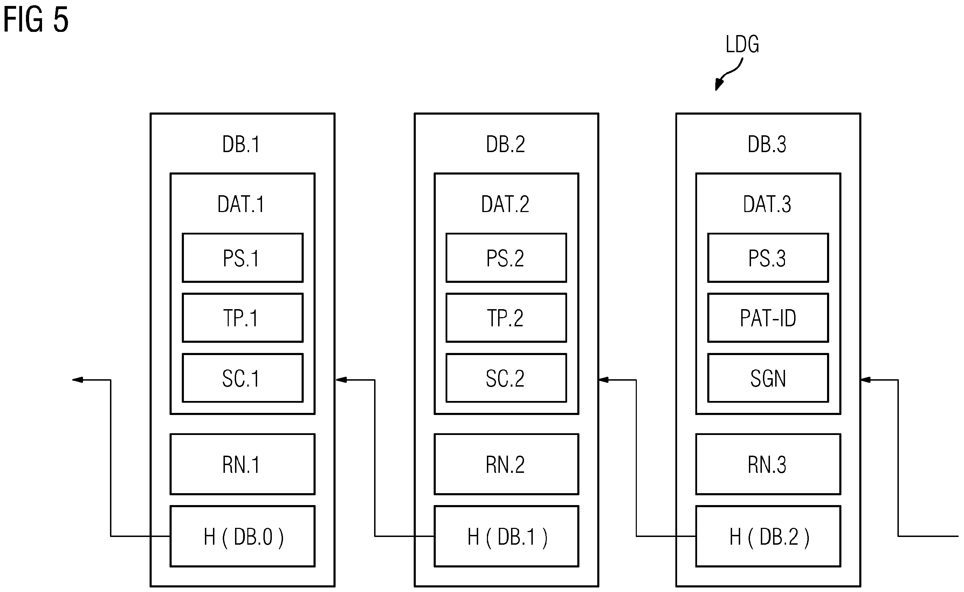

**Fig. 5** displays an embodiment of a distributed ledger LDG. In this embodiment, the distributed ledger LDG is a blockchain, which comprises data blocks DB.1, ..., DB.3 forming a chain. Alternatively, other distributed ledgers LDG can be used, for example blocktrees (where the data blocks DB.1, ..., DB.3 form a tree), or tangles (where the data blocks DB.1, ..., DB.3 form a directed acyclic graph), or other forms of linked data blocks.

In this embodiment, the distributed ledger LDG comprises data blocks DB.1, ..., DB.3, wherein each of the data blocks DB.1, ..., DB.3 is linked to another data block DB.0, ..., DB.2 (in this embodiment, the data block DB.1 is linked to a data block DB.1 which is not displayed). A link between two data blocks DB.1, ..., DB.3 is a directional information, this means that for each link there is a well-defined start of the link and a well-defined end of the link. In particular, if a first data block DB.1, ..., DB.3 is linked to a second data block DB.1, ..., DB.3, this means that the first data block DB.1, ..., DB.3 is the start of the link and the second data block DB.1, ..., DB.3 is the end of the link. In other words, a first data block DB.1, ..., DB.3 being linked to a second data block DB.1, ..., DB.3 does not imply that the second data block DB.1, ..., DB.3 is linked to the first data block DB.1, ..., DB.3.

In this embodiment, each data block DB.1, ..., DB.3 is linked to exactly one other data block DB.1, ..., DB.3. Alternatively, a data block DB.1, ..., DB.3 can be linked to several other data blocks DB.1, ..., DB.3. In particular, each data block DB.1, ..., DB.3 can be linked to a fixed number of other data blocks DB.1, ..., DB.3. A data block DB.1, ..., DB.3 can also be linked to itself.

In this embodiment, a first data block DB.1, ..., DB.3 is linked to a second data block DB.1, ..., DB.3 by the first data block DB.1, ..., DB.3 comprising a hash H(DB.0), ..., H(DB.3) of the second data block DB.1, ..., DB.3. Alternatively, other link information can be included into the first data block DB.1, ..., DB.3 to indicate that it is linked to the second data block DB.1, ..., DB.3. In this embodiment, the hash H(DB.0), ..., H(DB.3) is the result of the application of the SHA256 hash function on the second data block DB.1, ..., DB.3. In particular, the SHA256 hash function is applied to a concatenation of the contents of the second data block DB.1, ..., DB.3.

In particular, for inserting a data block DB.1, ..., DB.3 into the distributed ledger LDG, a consensus algorithm (e.g. proof of work, proof of storage, proof of stake, proof of elapsed time) must be executed, wherein the consensus algorithm may be based on choosing the nonce RN.1, ..., RN.3 of the data block DB.1, ..., DB.3 to be inserted. In particular, the nonce RN.1, ..., RN.3 is a data item (in particular a numbers) that can be chosen arbitrarily by the creator of the block. In this embodiment, the nonce RN.1, ..., RN.3 must be chosen by the creator of the data block DB.1, ..., DB.3 such that the hash H(DB.0), ..., H(DB.3) of said data block DB.1, ..., DB.3 fulfills a certain condition. In this embodiment, the condition is that the hash H(DB.0), ..., H(DB.3) of said data block DB.1, ..., DB.3 is smaller than a given threshold.

In this embodiment, each data block DB.1, ..., DB.3 comprises data DAT.1, ..., DAT.3. Here, the data blocks DB.1 and DB.2 comprise data DAT.1, DAT.2 each corresponding to an offer dataset OFF-DS, and the data block DB.3 comprises data DAT.3 corresponding to an order dataset ORD-DS. In general, the distributed ledger LDG can comprise further data blocks not comprising data relevant to the methods and systems of this invention. In other word, a multi-purpose distributed ledger LDG can be used, for example the "Bitcoin" blockchain or the "Ethereum" blockchain. Furthermore, each of the data blocks DB.1, ..., DB.3 of the distributed ledger LDG can comprise additional data, either data related to methods and systems of this invention, or data unrelated to methods and systems of this invention.

In particular, the data DAT.1, DAT.2 corresponding to an offer dataset OFF-DS were documented in the distributed ledger LDG by a requested provider entity R-PRVE or an offer providing system R-ORDS. In particular, the data DAT.3 corresponding to an order dataset ORD-DS was documented in the distributed ledger LDG by a payer entity PAYE or an order providing system ORDS.

In this embodiment, the data DAT.1, DAT.2 corresponding to an offer dataset OFF-DS comprises the procedure specification PS.1, PS.2, which was the basis for determining the offer dataset OFF-DS, a procedure-specific technical parameter TP.1, TP.2 and a smart contract SC.1, SC.2, the smart contract SC.1, SC.2 regulating the transfer of cryptocurrency if the result RES of the medical procedure was transmitted to the payer entity PAYE or to the order providing system ORDS.

The data DAT.1, DAT.2 corresponding to an offer dataset OFF-DS can be identical with the offer dataset OFF-DS, alternatively the offer dataset OFF-DS can comprise the data DAT.1, DAT.2 or the data DAT.1, DAT.2 can comprise the offer dataset OFF-DS.

In this embodiment, the data DAT.3 corresponding to an order dataset ORD-DS comprises a procedure specification PS.3, which was the basis for determining the selected provider entity S-PRVE, the identifier PAT-ID of the patient PAT being subject of the medical procedure, and a signature SGN signed with a private key of the payer entity PAYE or the order system ORDS, for example signing a dataset comprising an identifier of the selected provider entity S-PRVE (for example, a public key of the selected provider entity S-PRVE), the procedure specification PS.3 and the identifier PAT-ID of the patient PAT, in order to proof that the order was in fact issued or approved by the payer entity PAYE.

The data DAT.3 corresponding to an order dataset ORD-DS can be identical with the order dataset ORD-DS, alternatively the order dataset ORD-DS can comprise the data DAT.1, DAT.2 or the data DAT.1, DAT.2 can comprise the order dataset ORD-DS.

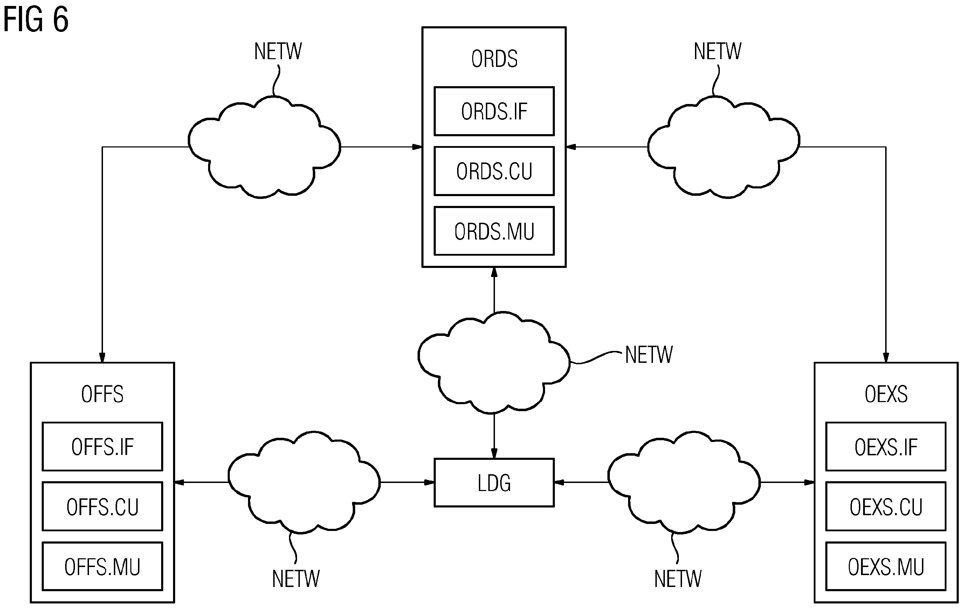

**Fig. 6** displays an order providing system ORDS, an offer providing system OFFS and an order execution system OEXS. In this embodiment, the offer providing system OFFS is different from the order execution system OEXS; alternatively, the offer providing system OFFS and the order execution system OEXS can be identical, as well as their sub-units.

The order providing system ORDS, the offer providing system OFFS and/or the order execution system OEXS may be a (personal) computer, a work-station, a virtual machine running on host hardware, a microcontroller, or an integrated circuit.

As an alternative, the order providing system ORDS, the offer providing system OFFS and/or the order execution system OEXS can be a real or a virtual group of computers (the technical term for a real group of computers is "cluster", the technical term for a virtual group of computers is "cloud"). The order providing system ORDS, the offer providing system OFFS and/or the order execution system OEXS comprise an interface ORDS.IF, OFFS.IF, OEXS.IF, a computation unit ORDS.CU, OFFS.CU, OEXS.CU and a memory unit ORDS.MU, OFFS.MU, OEXS.MU.

The order providing system ORDS is configured for executing the method for providing an order dataset ORD-DS. The offer providing system OFFS and/or the order execution system OEXS are configured for executing the method for providing an offer dataset OFF-DS. The order providing system ORDS, the offer providing system OFFS and/or the order execution system OEXS together are configured for executing the method for exchanging an order dataset ORD-DS and an offer dataset OFF-DS.

An interface ORDS.IF, OFFS.IF, OEXS.IF can be embodies as a hardware interface or as a software interface (e.g. PCI-Bus, USB or Firewire). In general, a computation unit ORDS.CU, OFFS.CU, OEXS.CU can comprise hardware elements and software elements, for example a microprocessor, a field programmable gate array (an acronym is "FPGA") or an application specific integrated circuit (an acronym is "ASIC"). A memory unit ORDS.MU, OFFS.MU, OEXS.MU can be embodied as non-permanent main memory (e.g. random access memory) or as permanent mass storage (e.g. hard disk, USB stick, SD card, solid state disk).

The order providing system ORDS and the offer providing system OFFS can exchange data and/or information via a network NETW, or they can exchange data and/or information by means of a distributed ledger LDG (possibly also involving the net-work NETW). The order providing system ORDS and the order execution system OEXS can exchange data and/or information via a network NETW, or they can exchange data and/or information by means of a distributed ledger LDG (possibly also involving the network NETW).

The network NETW can be realized as a LAN (acronym for "local area network"), in particular a WiFi network, or any other local connection, e.g. via Bluetooth or USB (acronym for "universal serial bus"). The network NETW can also be realized as a WAN (acronym for "wide area network"), in particular the network NETW can be identical with the internet. The network NETW can alternatively also be realized as a VPN (acronym for "virtual private net-work").

Wherever not already described explicitly, individual embodiments, or their individual aspects and features, can be combined or exchanged with one another without limiting or widening the scope of the described invention, whenever such a combination or exchange is meaningful and in the sense of this invention. Advantages which are described with respect to one embodiment of the present invention are, wherever applicable, also advantageous of other embodiments of the present invention.

## Claims

1. A computer-implemented method for providing an offer dataset (OFF-DS), comprising:
- receiving (REC-PS) a procedure specification (PS.1, ..., PS.3) related to a medical procedure from a payer entity (PAYE),
wherein the procedure specification (PS.1, ..., PS.3) comprises an procedure code of the medical procedure,
- identifying (IDFY-MA) a requested medical apparatus (R-APP) capable for executing the medical procedure based on the procedure code,
- determining (DET-TP) a procedure-specific technical parameter (TP.1, TP.2) of the requested medical apparatus (R-APP) based on the medical procedure and/or based on the procedure code,
- determining (DET-OFF-DS) the offer dataset (OFF-DS) based on the procedure-specific technical parameter (TP.1, TP.2), wherein the offer dataset (OFF-DS) is an offer dataset for the medical procedure, and
wherein the offer dataset (OFF-DS) comprises a smart contract (SC.1, SC.2),
- providing (PROV-OFF-DS) the offer dataset (OFF-DS) to the payer entity (PAYE), comprising documenting the offer dataset (OFF-DS) within a distributed ledger (LDG).

2. The method according to claim 1, wherein the procedure-specific technical parameter (TP.1, TP.2) relates to least one of:
- the procedure time necessary for the requested medical apparatus (R-APP) executing the medical procedure,
- the amount of consumables needed by the requested medical apparatus (R-APP) for executing the medical procedure,
- the number of operators necessary to execute the medical procedure with the requested medical apparatus (R-APP).

3. The method according to claim 1 or 2, wherein the procedure-specific technical parameter (TP.1, TP.2) is determined based on a database comprising at least one historic procedure-specific technical parameter of the requested medical apparatus (R-APP) executing a historic medical procedure related to the procedure code of the medical procedure.

4. The method according to one of the preceding claims, furthermore comprising:
- receiving (REC-ORD-DS) from the payer entity (PAYE) an order dataset (ORD-DS) comprising an procedure code of a medical procedure and an identifier (PAT-ID) of a patient (PAT),
- identifying (IDFY-PAT) the patient (PAT) based on the identifier (PAT-ID) of the patient (PAT),
- determining (DET-RES) a result (RES) of the medical procedure by executing the medical procedure on the patient (PAT) with a selected medical apparatus (S-APP),
- providing the result (PROV-RES) of the medical procedure to the payer entity (PAYE).

5. The method according to claim 4, wherein the identifier (PAT-ID) of the patient (PAT) is based on a public key corresponding to a private key,
wherein the private key is stored on a key device of the patient (PAT),
and wherein the identifying (IDFY-PAT) the patient (PAT) is based on an interaction with the key device of the patient (PAT).

6. The method according to claim 5, furthermore comprising:
- providing a digital signature (SGN) to the payer entity (PAYE), wherein the digital signature (SGN) is signed with the private key of the patient (PAT).

7. The method according to one of the preceding claims,
wherein the smart contract (SC.1, SC.2) regulates the transfer of cryptocurrency if a result (RES) of the medical procedure is provided to the payer entity (PAYE),
wherein the cryptocurrency is transferred from a first account corresponding to the payer entity (PAYE) to a second account corresponding to a provider entity (R-PRVE, S-PROVE).

8. A computer-implemented method for providing an order dataset (PROV-ORD-DS), comprising:
- optionally receiving an identifier of a patient (REC-PAT-ID),
- receiving an procedure code (REC-PC) of a medical procedure to be executed,
- determining at least one requested provider entity (DET-R-PRVE) capable for executing the medical procedure based on the procedure code REC-PC,
- receiving at least one offer dataset (REC-OFF-DS) based on the procedure code REC-PC,
wherein each of the at least one offer dataset (OFF-DS) is related to a provider entity of the at least one requested provider entities (R-PRVE),
- determining a selected provider entity (DET-S-PRVE) of the at least one requested provider entities (R-PRVE) based on the at least one offer dataset (OFF-DS),
- determining (DET-ORD-DS) the order dataset (ORD-DS) comprising the procedure code, the order dataset (ORD-DS) optionally comprising the identifier (PAT-ID) of the patient (PAT),
- providing (PROV-ORD-DS) the order dataset (ORD-DS) to the selected provider entity (S-PRVE).

9. The method according to claim 8, wherein the at least one offer dataset (OFF-DS) is documented in a distributed ledger LDG, and wherein receiving (REC-OFF-DS) the at least one offer dataset (OFF-DS) comprises querying the distributed ledger (LDG).

10. The method according to claim 8 or 9, wherein the at least one offer dataset (OFF-DS) comprises a smart contract (SC.1, SC.2).

11. An offer providing system (OFFS) for providing an offer dataset (OFF-DS), comprising:
- an interface (OFFS.IF), configured for receiving (REC-PS) a procedure specification (PS.1, ..., PS.3) related to a medical procedure from a payer entity (PAYE), wherein the procedure specification (PS.1, ..., PS.3) comprises an procedure code of the medical procedure,
furthermore configured for providing (PROV-OFF-DS) the offer dataset (OFF-DS) to the payer entity (PAYE), comprising documenting the offer dataset (OFF-DS) within a distributed ledger (LDG),
- an calculation unit (OFFS.CU), configured for identifying (IDFY-MA) a requested medical apparatus (R-APP) capable for executing the medical procedure based on the procedure code, furthermore configured for determining (DET-TP) a procedure-specific technical parameter (TP.1, TP.2) of the requested medical apparatus (R-APP) based on the medical procedure and/or based on the procedure code,
furthermore configured for determining (DET-OFF-DS) the offer dataset (OFF-DS) based on the procedure-specific technical parameter (TP.1, TP.2), wherein the offer dataset (OFF-DS) is an offer dataset for the medical procedure, and wherein the offer dataset (OFF-DS) comprises a smart contract (SC.1, SC.2) .

12. An order providing system (ORDS) for providing an order dataset (ORD-DS), comprising:
- an interface (ORDS.IF), optionally configured for receiving (REC-PAT-ID) an identifier (PAT-ID) of a patient (PAT), furthermore configured for receiving (REC-PC) an procedure code of a medical procedure to be executed,
furthermore configured for providing (PROV-ORD-DS) the order dataset (ORD-DS) to the selected provider entity (S-PRVE),
- a computation unit (ORDS.CU), configured for determining (DET-R-PRVE) at least one requested provider entity (R-PRVE) capable for executing the medical procedure based on the procedure code,
furthermore configured for receiving (REC-OFF-DS) at least one offer dataset (OFF-DS) based on the procedure code,
wherein each of the at least one offer dataset (OFF-DS) is related to a provider entity of the at least one requested provider entities (R-PRVE),
furthermore configured for determining (DET-S-PRVE) a selected provider entity (S-PRVE) of the at least one requested provider entities (R-PRVE) based on the at least one offer dataset (OFF-DS),
furthermore configured for determining (DET-ORD-DS) the order dataset (ORD-DS) comprising the procedure code, the order dataset (ORD-DS) optionally comprising the identifier (PAT-ID) of the patient (PAT).

13. A system comprising an offer providing system (OFFS) according to claim 11 and an order providing system (ORDS) according to claim 12.

14. A computer program
- comprising program elements which induce an offer providing system (OFFS) to carry out the steps of the method for providing an offer dataset (OFF-DS) according to one of the claims 1 to 7, when the program elements are loaded into a memory unit (OFFS.MU) of the offer providing system (OFFS),
- and/or comprising program elements which induce an order providing system (ORDS) to carry out the steps of the method for providing an order dataset (ORD-DS) according to one of the claims 8 to 10, when the program elements are loaded into a memory unit (ORDS.MU) of the order providing system (ORDS).

15. A computer-readable medium
- on which program elements are stored that can be read and executed by an offer providing system (OFFS), in order to perform steps of the method for providing an offer dataset (OFF-DS) according to one of the claims 1 to 7, when the program elements are executed by the offer providing system (OFFS),
- and/or on which program elements are stored that can be read and executed by an order providing system (ORDS), in order to perform steps of the method for providing an order dataset (ORD-DS) according to one of the claims 8 to 10, when the program elements are executed by the order providing system (ORDS).
